# EUROPEAN PATENT SPECIFICATION

(11) **EP 3 326 561 B1**
(45) Date of publication and mention of the grant of the patent: **01.07.2020**
(21) Application number: 15898981.4
(22) Date of filing: 21.07.2015
(51) Int. Cl.: A61B 18/12, A61B 18/14

(54) **BIPOLAR ELECTRODE FOR RADIO FREQUENCY ABLATION**
BIPOLARE ELEKTRODE FÜR RADIOFREQUENZABLATION
BIPOLAR ELECTRODE FOR RADIO FREQUENCY ABLATION

(43) Date of publication of application: 30.05.2018
(73) Proprietor: Starmed Co., Ltd., Gouang-si, Gyeonggi-do 410-722 (KR); Shin, Kyong-Min, Gyeonggi-do, 476-801 (KR)
(72) Inventor: SHIN, Kyong Min, Yangpyeong-gun Gyeonggi-do 476-801 (KR); SHIN, Kyung Hoon, Gimpo-si Gyeonggi-do 415-738 (KR); KIM, Dong Un, Gimpo-si Gyeonggi-do 415-090 (KR)
(74) Representative: Porta & Consulenti Associati S.p.A.
(86) International application number: PCT/KR2015/007529
(87) International publication number: WO 2017/014333

(56) References cited:
- WO-A1-2013/016203
- JP-A- 2012 161 603
- KR-A- 20130 140 954
- KR-B1- 101 342 088
- KR-B1- 101 415 900
- US-A- 164 184
- US-A- 3 901 242
- US-A1- 2008 097 429
- US-A1- 2012 203 217
- US-A1- 2015 025 525
- US-A1- 2015 025 525

## Description

### Technical Field

The present invention relates generally to a bipolar electrode for radio frequency ablation. More particularly, the present invention relates to a bipolar electrode for radio frequency ablation, wherein the electrode is configured to efficiently be moved to a vascular lesion such as varicose veins, and to control heat of the electrode to reduce a risk of thermal damage to surrounding tissue outside the lesion during treatment.

### Background Art

In general, cancerous tissue that develops in the body organs (liver, thyroid, etc.) is treated by surgical and non-surgical methods.

Recently, non-surgical methods have been widely used compared with surgical methods. There are well-known non-surgical methods such as transarterial chemoembolization, percutaneous ethanol injection, chemotherapy, and local hyperthermia, and among these, local hyperthermia is most effective.

Such local hyperthermia includes radiofrequency ablation, microwave cautery, laser cautery, etc. Among these, radiofrequency ablation is most effective and thus is highly demanded by physicians and patients.

Thus, as in Korean Patent KOKAI Publication No. 10-2013-0128926, a lesion such as cancer tissue or the like is heated by radio frequency emitted from an electrode and thus is cauterized and necrosed, or a vascular lesion such as varicose veins is heated whereby the inner walls of the veins are damaged, causing fibrosis of the blood vessels to ablate the swollen blood vessels.

However, physicians or patients are demanding an electrode that can efficiently be moved to the vascular lesion such as varicose veins, and of a more secure electrode that can reduce a risk of thermal damage to surrounding tissue outside the lesion due to heat of radiofrequency.
US 2008/0097429 describes an electrosurgical device which comprises an outer surface and includes a probe body, at least one conductor pair comprising a first electrode separated by a gap from a second electrode, and means in fluid communication with the lumen of a tube for distributing a fluid provided from the lumen of the tube to at least a portion of the surface of the electrosurgical device. In an embodiment, conductors are strip electrodes circumferentially directed and extending around the perimeter of the probe body in a spiral configuration. In another embodiment, conductors comprise three elongated longitudinally directed strip electrodes.

### Disclosure

### Technical Problem

The invention is defined in claim 1. Further aspects and preferred embodiments are defined in the dependent claim. Aspects, embodiments and examples of the present disclosure which do not fall under the scope of the appended claims do not form part of the invention and are merely provided for illustrative purposes. Accordingly, the present invention has been made keeping in mind the above problems occurring in the prior art, and an object of the present invention is to provide an electrode, wherein even when the electrode collides with the inner wall of a blood vessel when moving to a vascular lesion such as varicose veins, the electrode is efficiently moved to the lesion.

Another object of the present invention is to provide an electrode capable of controlling a heat generation range of radiofrequency whereby a risk of thermal damage to surrounding tissue outside the lesion during treatment.

### Technical Solution

In order to accomplish the above object, according to one aspect of the present invention, there is provided a bipolar electrode for radio frequency ablation, the electrode including: a cylindrical body provided with first and second coupling grooves formed on an outer circumferential surface of the cylindrical body in a screw thread shape and spaced apart from each other at a predetermined interval; an active electrode body wound on the first coupling groove of the cylindrical body a plurality of times to be inserted thereinto, and connected to a first terminal of a radiofrequency generator; and a passive electrode body wound on the second coupling groove of the cylindrical body a plurality of times to be inserted thereinto, and connected to a second terminal of the radiofrequency generator.

According to another aspect of the present invention, there is provided a bipolar electrode for radio frequency ablation, the electrode including: a cylindrical body provided with first and second coupling grooves formed on an outer circumferential surface of the cylindrical body in a screw thread shape and spaced apart from each other at a predetermined interval, and provided therein with a cooling water circulation channel in which cooling water supplied from a cooling pipe circulates; an active electrode body wound on the first coupling groove of the cylindrical body a plurality of times to be inserted thereinto, and connected to a first terminal of a radiofrequency generator; and a passive electrode body wound on the second coupling groove of the cylindrical body a plurality of times to be inserted thereinto, and connected to a second terminal of the radiofrequency generator.

### Advantageous Effects

As described above, according to the present invention, the active electrode body and the passive electrode body that emit radiofrequency heat are inserted into the screw thread-shaped first and second coupling grooves formed on the outer circumferential surface of the cylindrical body, whereby even when the electrode collides with the inner wall of a blood vessel when moving, it is possible to prevent the active electrode body and the passive electrode body from being separated from the first and second coupling grooves.

Further, according to the present invention, the outer circumferential surface of the cylindrical body, in which the active electrode body and the passive electrode body are inserted into the first and second coupling grooves, is smooth, whereby it is possible to prevent the electrode from being interrupted by the inner wall of the blood vessel when moving to a lesion.

Further, according to the present invention, the first and second coupling grooves are formed by extending on the outer circumferential surface of the cylindrical body in the lengthwise direction thereof, whereby the active electrode body and the passive electrode body can be quickly inserted into the first and second coupling grooves.

Further, according to the present invention, the cooling water circulation channel in which cooling water circulates is formed in the cylindrical body, whereby it is possible to remove heat of radiofrequency to thereby control a heat generation range of the electrode, and it is possible to achieve a reduced risk of thermal damage to surrounding tissue outside the lesion.

### Description of Drawings

FIG. 1 is a view showing a device provided with a bipolar electrode for radio frequency ablation according to first and second embodiments of the present invention.
FIGS. 2 and 3 are views showing the bipolar electrode for radio frequency ablation according to the first embodiment of the present invention.
FIGS. 4 and 5 are views showing the bipolar electrode for radio frequency ablation according to the second embodiment of the present invention.
FIG. 6 is a view showing a device provided with a bipolar electrode for radio frequency ablation according to third and fourth embodiments of the present invention.
FIGS. 7 to 9 are views showing the bipolar electrode for radio frequency ablation according to the third embodiment of the present invention.
FIGS. 10 to 12 are views showing the bipolar electrode for radio frequency ablation according to the fourth embodiment of the present invention.

### Best Mode

Hereinafter, exemplary embodiments of the present invention will now be described in detail with reference to the accompanying drawings.

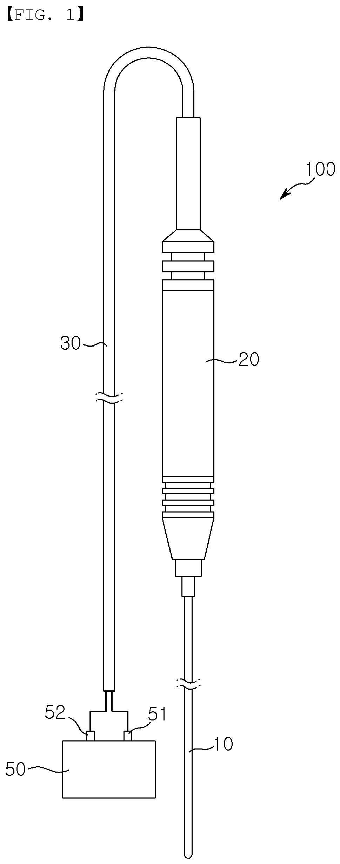

As shown in FIG. 1, an electrode device 100 for radio frequency ablation to which an electrode 10 according to first and second embodiments of the present invention is applied includes an electrode 10, a handle 20, an electrode wire 30, and a radiofrequency generator (50).

The handle 20, which is positioned at a rear end of the electrode 10,is a part that is gripped by an operator who wants to use the electrode 10 and, the electrode wire 30 electrically connects the electrode 10 and the radiofrequency generator 50 through the handle 20, and the radiofrequency generator 50 is a device for generating a radiofrequency alternating current and is configured such that a positive terminal and a negative terminal are selectively connected to the active electrode body 2 or the passive electrode body 3 to supply the radiofrequency alternating current to the electrode 10.

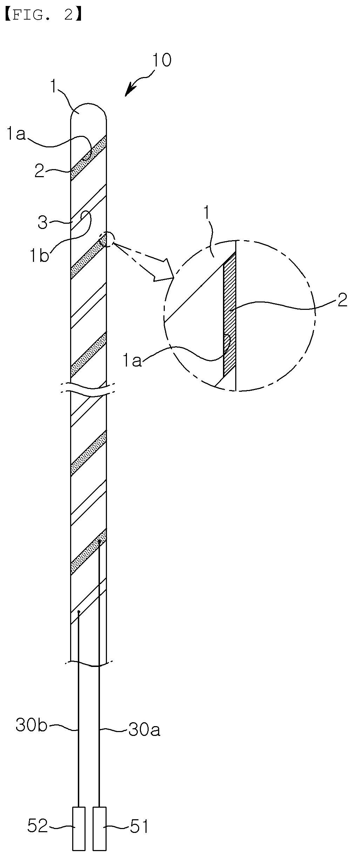

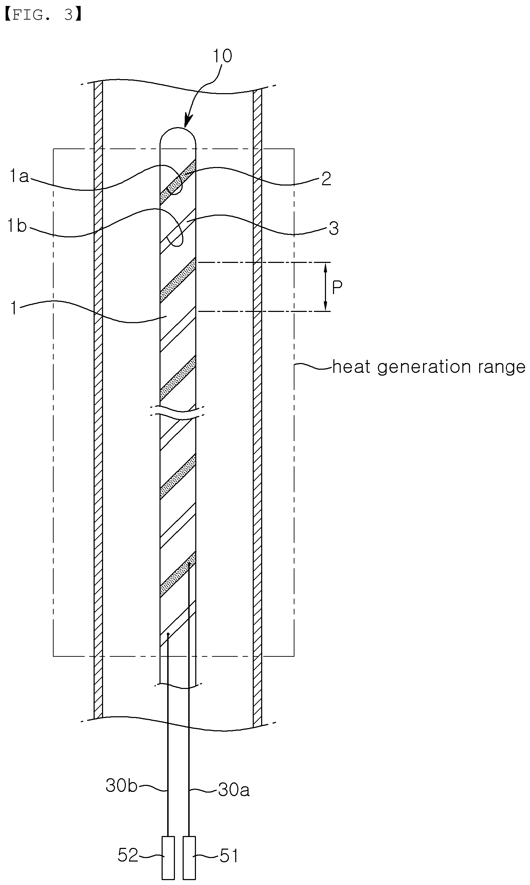

As shown in FIGS. 2 and 3, the electrode 10 according to the first embodiment of the present invention includes: a cylindrical body 1 provided with first and second coupling grooves 1a and 1b formed on an outer circumferential surface of the cylindrical body in a screw thread shape and spaced apart from each other at a predetermined interval; the active electrode body 2 wound on the first coupling groove 1a of the cylindrical body 1 a plurality of times to be inserted thereinto and connected to a first terminal of the radiofrequency generator 50; and the passive electrode body 3 wound on the coupling groove 1b of the cylindrical body 1 a plurality of times to be inserted thereinto and connected to a second terminal of the radiofrequency generator 50.

Herein, the cylindrical body 1 has a circular shape at a front end thereof such that the cylindrical body is efficiently inserted into a blood vessel such as a vein, and the cylindrical body is connected to an end portion of a moving wire when applied to a catheter.

Herein, the cylindrical body 1 is made of an insulating material.

Further, as shown in one side of the electrode 10, the first and second coupling grooves 1a and 1b are configured such that a plate having a parallelogram shape in cross section is grooved along the outer circumferential surface of the cylindrical body 1 in a screw thread shape. The active electrode body 2 and the passive electrode body 3 have a parallelogram shape in cross section, thereby being efficiently inserted into the first and second coupling grooves 1a and 1b.

Accordingly, the active electrode body 2 and the passive electrode body 3 are inclindly wound on the first and second coupling grooves 1a and 1b in a screw thread direction to be inserted thereinto without protruding from the outer circumferential surface of the cylindrical body 1.

In other words, when a vascular lesion such as varicose veins is treated, the electrode 10 can be prevented from being interrupted by the inner wall of a blood vessel such as a vein. At the same time, even when the electrode collides with the inner wall of the blood vessel, the active electrode body 2 and the passive electrode body 3 can be prevented from being separated from the first and second coupling grooves 1a and 1b.

The active electrode body 2 and the passive electrode body 3 are parts to emit radiofrequency current generated from the radiofrequency generator 50 from the electrode 10, wherein the active electrode body 2 is connected to an active terminal 51 of the radiofrequency generator 50 via an active wire 30a of the electrode wire 30, and the passive electrode body 3 is connected to a passive terminal 52 of the radiofrequency generator 30 via a passive wire 30b of the electrode wire 30. Herein, the active terminal 51 or the passive terminal 52 may be a positive terminal or a negative terminal in accordance with convenience.

In particular, when the active electrode body 2 and the passive electrode body 3 emit radiofrequency energy in a state in which the active electrode body and the passive electrode body maintain a predetermined interval therebetween, heat generation begins around an intermediate point of a pitch P of the active electrode body 2 and the passive electrode body 3. As shown in FIG. 3, a heat generation range has a rectangular cylindrical shape surrounding the cylindrical body 1.

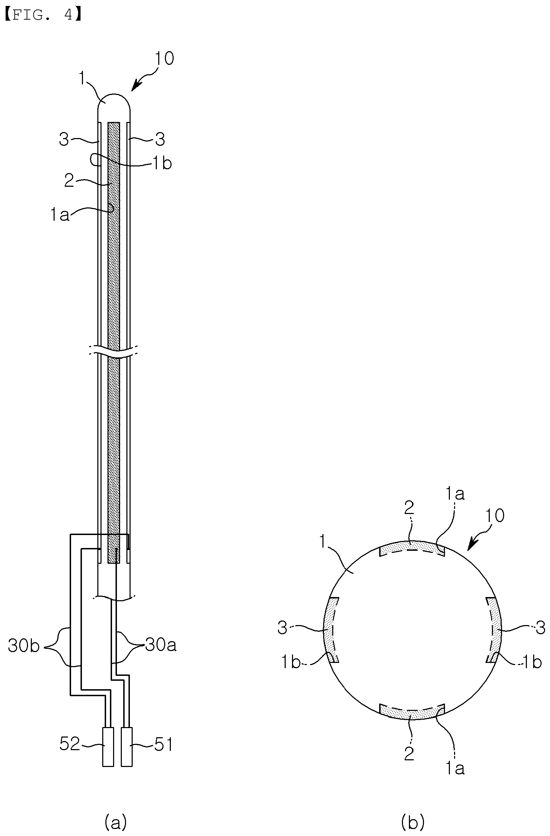

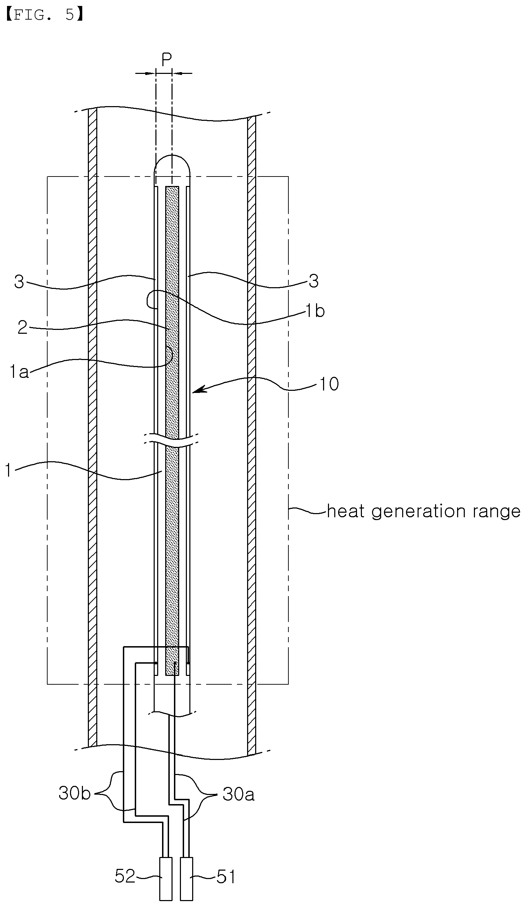

As shown in FIGS. 4 and 5, the electrode 10 according to the second embodiment of the present invention includes: a cylindrical body 1 provided with first and second coupling grooves 1a and 1b formed by extending on an outer circumferential surface of the cylindrical body in a lengthwise direction thereof and spaced apart from each other at a predetermined interval; an active electrode body 2 inserted into the first coupling groove 1a of the cylindrical body 1 and connected to a first terminal of a radiofrequency generator 50; and a passive electrode body 3 inserted into the second coupling groove 1b of the cylindrical body 2 and connected to a second terminal of the radiofrequency generator 50.

Herein, the cylindrical body 1 has a circular shape at a front end thereof such that the cylindrical body is efficiently inserted into a blood vessel such as a vein, and the cylindrical body is connected to an end portion of a moving wire when applied to a catheter.

Herein, the cylindrical body 1 is made of an insulating material.

Further, as shown in one side of the electrode 10, the first and second coupling grooves 1a and 1b are grooved in a rectangular shape by extending on the outer circumferential surface of the cylindrical body 1 in the lengthwise direction thereof, and the active electrode body 2 and the passive electrode body 3 have a rectangular shape such that the active electrode body and the passive electrode body are efficiently inserted into the first and second coupling grooves 1a and 1b of the cylindrical body 1.

Herein, as seen from the plane of the electrode 10, the first and second coupling grooves 1a and 1b are grooved in a curved shape such that curved surfaces are curved outwards from the cylindrical body 1, and the active electrode body 2 and the passive electrode body 3 are formed in a curved shape such that inner and outer surfaces are curved outwards from the cylindrical body.

Further, the first coupling groove 1a is formed at opposite first and second sides of the outer circumferential surface of the cylindrical body 1, and the second coupling groove 1b is formed on the outer circumferential surface of the cylindrical body 1 in a pair at positions orthogonal to the first coupling groove 1a.

Accordingly, the active electrode body 2 and the passive electrode body 3 are inserted into the first and second coupling grooves 1a and 1b without protruding from the outer circumferential surface of the cylindrical body 1.

In other words, when a vascular lesion such as varicose veins is treated, the electrode 10 can be prevented from being interrupted by the inner wall of the blood vessel such as a vein. At the same time, even when the electrode collides with the inner wall of the blood vessel, the active electrode body 2 and the passive electrode body 3 can be prevented from being separated from the first and second coupling grooves 1a and 1b.

In addition, since the active electrode body 2 and the passive electrode body 3 according to the second embodiment of the present invention are formed in a rectangular shape having the curved inner and outer surfaces, the active electrode body and the passive electrode body can be quickly inserted into the rectangular first and second coupling grooves 1a and 1b each having the curved surface, compared with the active electrode body 2 and the passive electrode body 3 according to the first embodiment of the present invention, which are inserted into the first and second coupling grooves 1a and 1b in a screw thread shape.

Herein, the active electrode body 2 and the passive electrode body 3 are connected to the active terminal 51 and the passive terminal 52 of the radiofrequency generator 50, respectively, as in the first embodiment of the present invention, wherein the active terminal 51 or the passive terminal 52 may be a positive terminal or a negative terminal in accordance with convenience.

Further, when the active electrode body 2 and the passive electrode body 3 emit radiofrequency energy in a state in which the active electrode body and the passive electrode body maintain a predetermined interval therebetween, heat generation begins around an intermediate point of a pitch P of the active electrode body 2 and the passive electrode body 3. As shown in FIG. 5, the heat generation range has a rectangular cylindrical shape surrounding the cylindrical body 1 as in the first embodiment of the present invention.

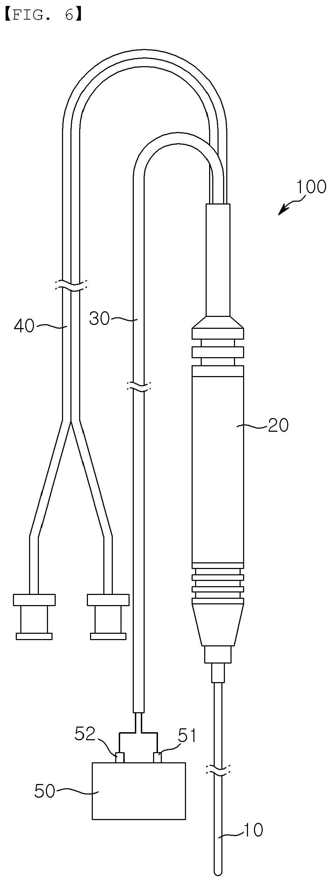

As shown in FIG. 6, an electrode device for radio frequency ablation 100 to which an electrode 10 according to third and fourth embodiments of the present invention is applied includes an electrode 10, a handle 20, an electrode wire 30, a cooling pipe 40, and a radiofrequency generator 50.

The handle 20, which is positioned at a rear end of the electrode 10, is a part that is gripped by an operator who wants to use the electrode 10, the electrode wire 30 electrically connects the electrode 10 and the radiofrequency generator 50 through the handle 20, the cooling pipe 40 is connected to the handle 20 such that the cooling pipe supplies and recovers cooling water to the electrode 10 to circulates the cooling water, and the radiofrequency generator 50 is a device for generating a radiofrequency alternating current and is configured such that a positive terminal and a negative terminal are selectively connected to the active electrode body 2 or the passive electrode body 3 to supply the radiofrequency alternating current to the electrode 10.

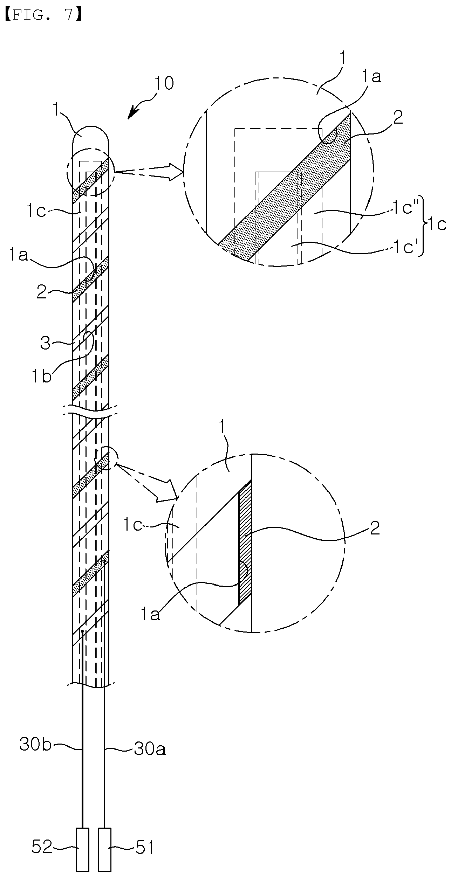

As shown in FIGS. 7 to 9, the electrode 10 according to the third embodiment of the present invention includes: a cylindrical body 1 provided with first and second coupling grooves 1a and 1b formed on an outer circumferential surface of the cylindrical body in a screw thread shape and spaced apart from each other at a predetermined interval, and provided therein with a cooling water circulation channel 1c in which cooling water supplied from a cooling pipe 40 circulates; an active electrode body 2 wound on the first coupling grooves 1a of the cylindrical body 1 a plurality of times to be inserted thereinto and connected to a first terminal of a radiofrequency generator 50; and a passive electrode body 30 wound on the second coupling groove 1b of the cylindrical body 1 a plurality of times to be inserted thereinto and connected to a second terminal of the radiofrequency generator 50.

Herein, the cylindrical body 1 has a circular shape at a front end thereof such that the cylindrical body is efficiently inserted into a blood vessel such as a vein, and the cylindrical body is connected to an end portion of a moving wire when applied to a catheter.

In addition, the cylindrical body 1 is made of an insulating material, and the cooling water circulation channel 1c includes a cylindrical inflow channel 1c' into which cooling water supplied from the cooling pipe 40 flows, and a cylindrical outflow channel 1c" provided outside the inflow passage 1c' and discharging the cooling water flowing in from the inflow passage 1c' to the cooling pipe 40.

Herein, the outflow channel 1c" and the inflow channel 1c' are formed by extending to the front end of the cylindrical body 1. In addition, the outflow channel 1c" has a length longer than that of the inflow channel 1c', so that cooling water flowing into the inflow channel 1c' moves from the front end to a rear end of the cylindrical body 1 through the outflow channel 1c" and is then recovered again to the cooling pipe 40.

Meanwhile, the active electrode body 2, the passive electrode body 3, and the first and second coupling grooves 1a and 1b have the same shapes as those of the active electrode body 2, the passive electrode body 3, and the first and second coupling grooves 1a and 1b according to the first embodiment of the present invention, respectively.

Accordingly, the active electrode body 2 and the passive electrode body 3 are inclindly wound on the first and second coupling grooves 1a and 1b in a screw thread direction to be inserted thereinto without protruding from the outer circumferential surface of the cylindrical body 1.

In other words, when a vascular lesion such as varicose veins is treated, the electrode 10 can be prevented from being interrupted by the inner wall of the blood vessel such as a vein. At the same time, even when the electrode collides with the inner wall of the blood vessel, the active electrode body 2 and the passive electrode body 3 can be prevented from being separated from the first and second coupling grooves 1a and 1b.

Further, the active electrode body 2 and the passive electrode body 3 are parts to emit radiofrequency current generated from the radiofrequency generator 50 from the electrode 10, wherein the active electrode body 2 is connected to an active terminal 51 of the radiofrequency generator 50 via an active wire 30a of the electrode wire 30, and the passive electrode body 3 is connected to a passive terminal 52 of the radiofrequency generator 30 via a passive wire 30b of the electrode wire 30. Herein, the active terminal 51 or the passive terminal 52 may be a positive terminal or a negative terminal in accordance with convenience.

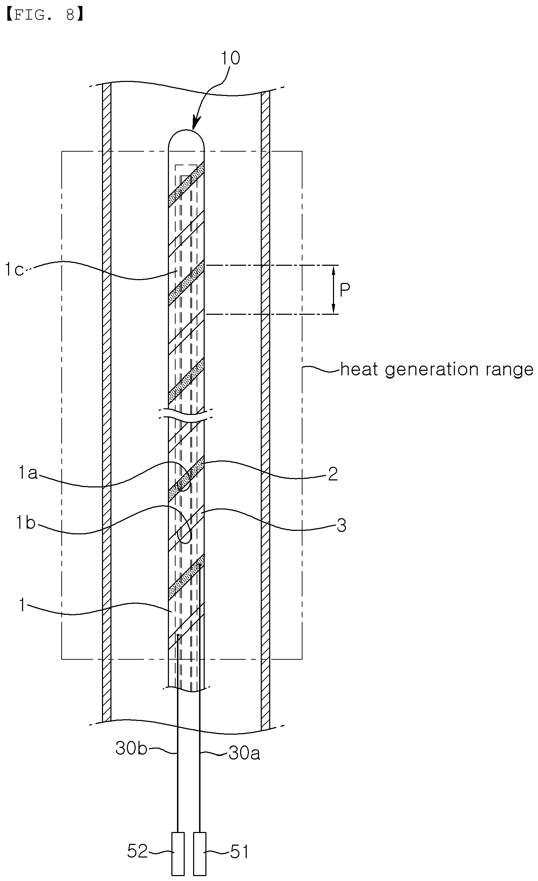

In particular, when the active electrode body 2 and the passive electrode body 3 emit radiofrequency energy in a state in which the active electrode body and the passive electrode body maintain a predetermined interval therebetween, heat generation begins around an intermediate point of a pitch P of the active electrode body 2 and the passive electrode body 3. As shown in FIG. 8, a heat generation range has a rectangular cylindrical shape surrounding the cylindrical body 1.

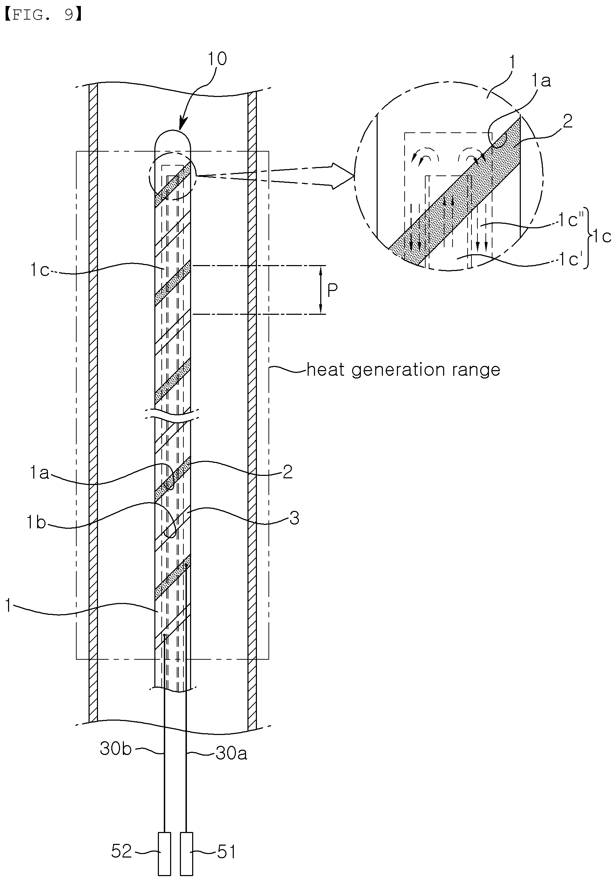

In addition, as shown in FIG. 9, cooling water circulates in the cooling water circulation channel 1c to remove heat of the electrode 10 from which radiofrequency energy is emitted. Thus, it is possible to achieve more precise control of a heat generation range of the electrode 10 than the first and second embodiments of the present invention, and to reduce a risk of thermal damage to a region other than the vascular lesion such as varicose veins.

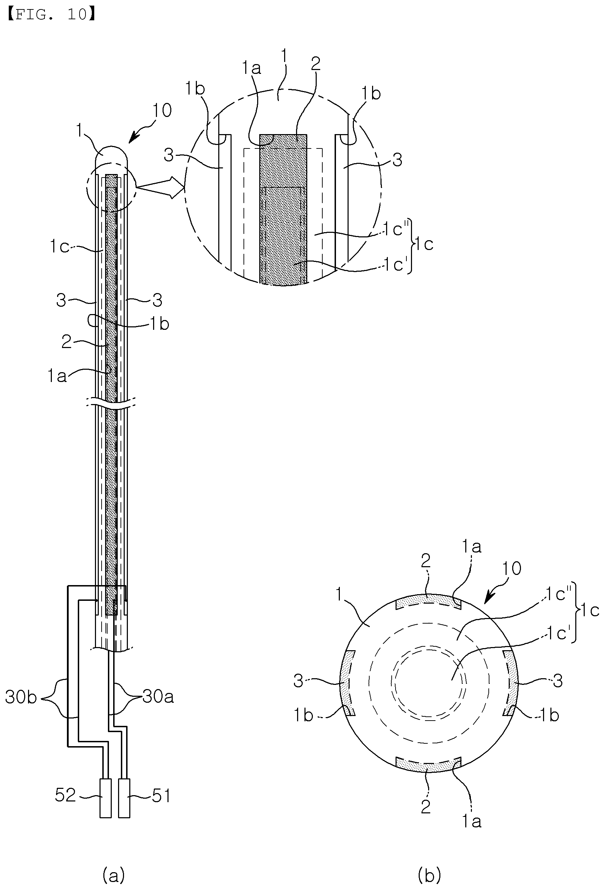

As shown in FIGS. 10 to 12, the electrode 10 according to the fourth embodiment of the present invention includes: a cylindrical body 1 provided with first and second coupling grooves 1a and 1b formed by extending on an outer circumferential surface of the cylindrical body in a lengthwise direction thereof and spaced apart from each other at a predetermined interval, and provided therein with a cooling water circulation channel 1c in which cooling water supplied from the cooling pipe 40 circulates; an active electrode body 2 inserted into the first coupling groove 1a of the cylindrical body 1 and connected to a first terminal of a radiofrequency generator 50; and a passive electrode body 3 inserted into the second coupling groove 1b of the cylindrical body 2 and connected to a second terminal of the radiofrequency generator 50.

Herein, the cylindrical body 1 has a circular shape at a front end thereof such that the cylindrical body is efficiently inserted into a blood vessel such as a vein, and the cylindrical body is connected to an end portion of a moving wire when applied to a catheter.

In addition, the cylindrical body 1 is made of an insulating material, and the cooling water circulation channel 1c includes a cylindrical inflow channel 1c' into which cooling water supplied from the cooling pipe 40 flows, and a cylindrical outflow channel 1c" provided outside the inflow passage 1c' and discharging the cooling water flowing in from the inflow passage 1c' to the cooling pipe 40.

Herein, the outflow channel 1c" and the inflow channel 1c' are formed by extending to the front end of the cylindrical body 1. In addition, the outflow channel 1c" has a length longer than that of the inflow channel 1c', so that cooling water flowing into the inflow channel 1c' moves from the front end to a rear end of the cylindrical body 1 through the outflow channel 1c" and is then recovered again to the cooling pipe 40.

Meanwhile, the active electrode body 2, the passive electrode body 3, and the first and second coupling grooves 1a and 1b have the same shapes as those of the active electrode body 2, the passive electrode body 3, and the first and second coupling grooves 1a and 1b according to the second embodiment of the present invention, respectively.

Accordingly, the active electrode body 2 and the passive electrode body 3 are inserted into the first and second coupling grooves 1a and 1b without protruding from the outer circumferential surface of the cylindrical body 1.

In other words, when a vascular lesion such as varicose veins is treated, the electrode 10 can be prevented from being interrupted by the inner wall of the blood vessel such as a vein. At the same time, even when the electrode collides with the inner wall of the blood vessel, the active electrode body 2 and the passive electrode body 3 can be prevented from being separated from the first and second coupling grooves 1a and 1b.

In addition, since the active electrode body 2 and the passive electrode body 3 according to the fourth embodiment of the present invention are formed in a rectangular shape having the curved inner and outer surfaces, the active electrode body and the passive electrode body can be quickly inserted into the rectangular first and second coupling grooves 1a and 1b each having the curved surface, compared with the active electrode body 2 and the passive electrode body 3 according to the third embodiment of the present invention, which are inserted into the first and second coupling grooves 1a and 1b in a screw thread shape.

Herein, the active electrode body 2 and the passive electrode body 3 are connected to the active terminal 51 and the passive terminal 52 of the radiofrequency generator 50, respectively, as in the third embodiment of the present invention, wherein the active terminal 51 or the passive terminal 52 may be a positive terminal or a negative terminal in accordance with convenience.

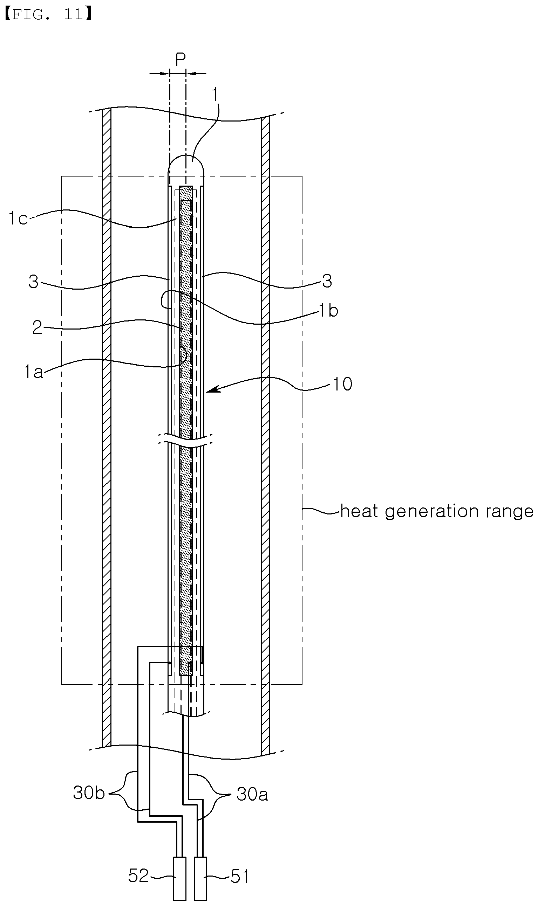

Further, when the active electrode body 2 and the passive electrode body 3 emit radiofrequency energy in a state in which the active electrode body and the passive electrode body maintain a predetermined interval therebetween, heat generation begins around an intermediate point of a pitch P of the active electrode body 2 and the passive electrode body 3. As shown in FIG. 11, a heat generation range has a rectangular cylindrical shape surrounding the cylindrical body 1 as in the third embodiment of the present invention.

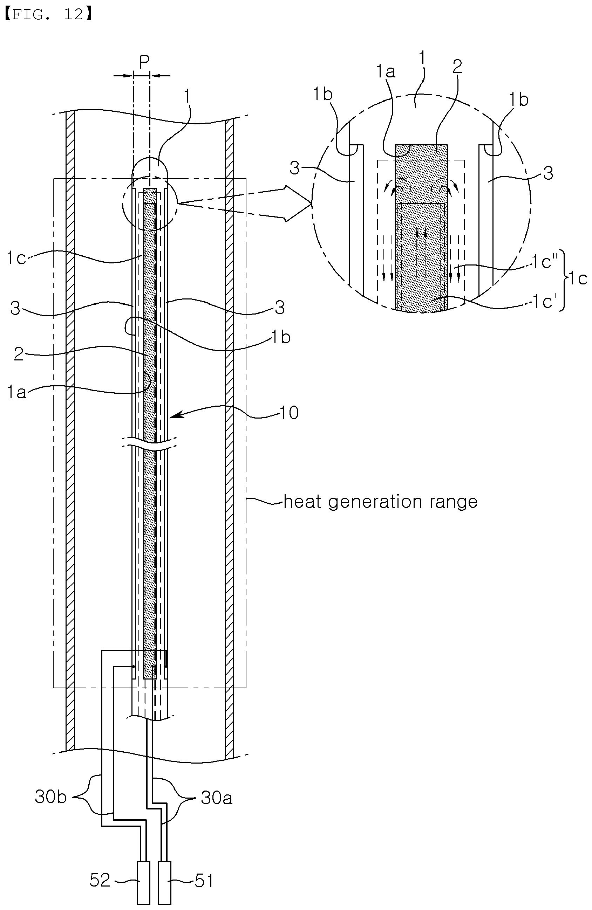

In addition, as shown in FIG. 12, cooling water circulates in the cooling water circulation channel 1c to remove heat of the electrode 10 from which radiofrequency energy is emitted as in the third embodiment of the present invention. Thus, it is possible to achieve more precise control of a heat generation range of the electrode 10 than the first and second embodiments of the present invention, and to reduce a risk of thermal damage to a region other than the vascular lesion such as varicose veins.

The operation and effect of the bipolar electrode 10 for radio frequency ablation of the present invention will be described as follows.

As shown in FIGS. 1 to 3, the electrode 10 according to the first embodiment of the present invention is inserted into a blood vessel upon start of treatment in a vascular lesion such as varicose veins, moved by the electrode device 100 to locate the lesion, and placed at a correct target position, that is, the lesion, by a mark means such as an X-ray marker.

Herein, the active electrode body 2 and the passive electrode body 3 are inclindly wound on the first and second coupling grooves 1a and 1b in the screw thread direction to be inserted thereinto without protruding from the outer circumferential surface of the cylindrical body 1 such that the outer circumferential surface of the cylindrical body 1 is smooth. Accordingly, even when the electrode 10 collides with the inner wall of the blood vessel such as a vein while moving, the electrode 10 can be moved to the lesion without being interrupted by the inner wall of the blood vessel.

In other words, since the electrode 10 can be prevented from being interrupted by the inner wall of the blood vessel such as a vein, the electrode can be quickly placed on the lesion without delay due to interruption.

In other words, even when the active electrode body 2 and the passive electrode body 3 collide with the inner wall of the blood vessel such as a vein, it is possible to prevent separation from the cylindrical body 1. Consequently, it is not necessary to replace the electrode 10 due to separation during treatment, and it is possible to prevent undesirable radiofrequency heat from being generated when the active electrode body 2 and the passive electrode body 3 are displaced due to the separation.

Thereafter, when placement of the electrode 10 is confirmed, the radiofrequency generator 50 operates such that radiofrequency alternating current is emitted between the active electrode body 2 and the passive electrode body 3. Herein, the active electrode body 2 and the passive electrode body 3 are configured such that radiofrequency energy is emitted between respective adjacent electrode bodies that are spaced apart from each other at a pitch P interval, thereby forming a radiofrequency energy emission region in a cylindrical shape.

Accordingly, the temperature of lesion tissue is increased by heat of the radiofrequency energy, whereby the vascular lesion such as varicose veins can be treated.

As shown in FIGS. 1, 4, and 5, the electrode 10 according to the second embodiment of the present invention is placed on the vascular lesion such as varicose veins, as in the first embodiment of the present invention.

Herein, the active electrode body 2 and the passive electrode body 3 according to the first embodiment of the present invention is not changed in shape, and the active electrode body 2 and the passive electrode body 3 are configured to be quickly inserted into the first and second coupling grooves 1a and 1b without protruding from the outer circumferential surface of the cylindrical body 1 without any change in shape. Accordingly, even when the electrode 10 collides with the inner wall of the blood vessel such as a vein when moving, the electrode 10 can move a lesion without interruption.

In other words, the electrode 10 according to the second embodiment of the present invention can be quickly placed on the lesion as in the first embodiment of the present invention.

In other words, even when the active electrode body 2 and the passive electrode body 3 collide with the inner wall of the blood vessel such as a vein, it is possible to prevent separation from the cylindrical body 1. Consequently, it is not necessary to replace the electrode 10 due to separation during treatment, and it is possible to prevent undesirable radiofrequency heat from being generated when the active electrode body 2 and the passive electrode body 3 are displaced due to the separation.

Accordingly, the temperature of lesion tissue is increased by heat of the radiofrequency energy, whereby a vascular lesion such as varicose veins can be treated.

In addition, even when the active electrode body 2 and the passive electrode body 3 are separated from the cylindrical body 1 by colliding with the inner wall of the blood vessel such as a vein, the active electrode body and the passive electrode body can be more efficiently inserted into the first and second coupling grooves 1a and 1b compared with the first embodiment of the present invention, thereby enabling prompt restarting of treatment.

As shown in FIGS. 6 to 9, the electrode 10 according to the third embodiment of the present invention is inserted into a blood vessel upon start of treatment in a vascular lesion such as varicose veins, moved by the electrode device 100 to locate the lesion, and placed at a correct target position, that is, the lesion, by a mark means such as an X-ray marker.

Herein, since the electrode 10 is prevented from being interrupted by the inner wall of the blood vessel such as a vein as in the other embodiments, the electrode can be quickly placed on the lesion to emit radiofrequency energy to the lesion.

Thereafter, when placement of the electrode 10 is confirmed, the radiofrequency generator 50 operates such that radiofrequency alternating current is emitted between the active electrode body 2 and the passive electrode body 3. Herein, the active electrode body 2 and the passive electrode body 3 are configured such that radiofrequency energy is emitted between respective adjacent electrode bodies that are spaced apart from each other at a pitch P interval, thereby forming a radiofrequency energy emission region in a cylindrical shape.

Thus, as shown in FIG. 9, cooling water circulates in the cooling water circulation channel 1c to remove heat of the electrode 10 that emits radiofrequency energy, thereby controlling a heat generation range of the electrode 10.

In other words, by controlling a heat generation range of radiofrequency energy of the electrode 10, it is possible to minimize the thickness of the lesion, that is, a risk of thermal damage to surrounding tissue can be reduced compared with the first and second embodiments of the present invention.

In addition, since heat of the active electrode body 2 and the passive electrode body 3 can be quickly removed with cooling water circulating in the cooling water circulation channel 1c, it is possible to achieve reduced time required for treatment in the lesion such as varicose veins, and to achieve reduced physical burden on a patient due to long-term treatment.

Moreover, cooling water circulating in the cooling water circulation channel 1c flows into the inflow channel 1c' and flows from the front end of the cylindrical body 1 to the rear end of the cylindrical body 1 through the outflow channel 1c", whereby time that cooling water stays in the cooling water circulation channel 1c can be prolonged.

In other words, it is possible to maintain a constant heat generation range of radiofrequency energy of the electrode 10.

Furthermore, it is possible to delay time that the heat generation range of radiofrequency energy of the electrode 10 is changed due to disruption of supply of the cooling water, and to gain time to resupply the cooling water.

As shown in FIGS. 6, and FIGS. 10 to 12, the electrode 10 according to the fourth embodiment of the present invention is placed on a vascular lesion such as varicose veins as in the third embodiment of the present invention.

Herein, since the electrode 10 is prevented from being interrupted by the inner wall of the blood vessel such as a vein as in the other embodiments, the electrode can be quickly placed on the lesion to emit radiofrequency energy to the lesion.

Thereafter, when placement of the electrode 10 is confirmed, the radiofrequency generator 50 operates such that radiofrequency alternating current is emitted between the active electrode body 2 and the passive electrode body 3. Herein, the active electrode body 2 and the passive electrode body 3 are configured such that the radiofrequency energy is emitted between respective adjacent electrode bodies that are spaced apart from each other at a pitch P interval, thereby forming a radiofrequency energy emission region in a cylindrical shape.

Herein, as shown in FIG. 12, cooling water circulates in the cooling water circulation channel 1c to remove heat of the electrode 10 that emits radiofrequency energy, thereby controlling a heat generation range of the electrode 10.

In other words, by controlling a heat generation range of radiofrequency energy of the electrode 10, it is possible to minimize the thickness of the lesion, that is, a risk of thermal damage to surrounding tissue can be reduced compared with the first and second embodiments of the present invention.

In other words, since heat of the active electrode body 2 and the passive electrode body 3 can be quickly removed with cooling water circulating in the cooling water circulation channel 1c, it is possible to achieve reduced time required for treatment in the lesion such as varicose veins, and to achieve reduced physical burden on a patient due to long-term treatment.

In addition, even when the active electrode body 2 and the passive electrode body 3 are separated from the cylindrical body 1 by colliding with the inner wall of the blood vessel such as a vein, the active electrode body and the passive electrode body can be more efficiently inserted into the first and second coupling grooves 1a and 1b compared with the third embodiment of the present invention, thereby enabling prompt restarting of treatment.

Moreover, cooling water circulating in the cooling water circulation channel 1c flows into the inflow channel 1c' and flows from the front end of the cylindrical body 1 to the rear end of the cylindrical body 1 through the outflow channel 1c", whereby time that cooling water stays in the cooling water circulation channel 1c can be prolonged.

In other words, it is possible to maintain a constant heat generation range of radiofrequency energy of the electrode 10.

Furthermore, it is possible to delay time that the heat generation range of radiofrequency energy of the electrode 10 is changed due to disruption of supply of the cooling water, and to gain time to resupply the cooling water.

Although a preferred embodiment of the present invention has been described for illustrative purposes, those skilled in the art will appreciate that various modifications, additions and substitutions are possible, without departing from the scope of the invention as disclosed in the accompanying claims.

### <Description of the Reference Numerals in the Drawings>

1 : cylindrical body
1a : first coupling groove
1b : second coupling groove
1c : cooling water circulation channel
2 : active electrode body
3 : passive electrode body
10 : bipolar electrode for radio frequency ablation
20 : handle 30 : electrode wire
40 : cooling pipe
50 : radiofrequency generator
100 : bipolar electrode device for radio frequency ablation

## Claims

1. A bipolar electrode (10) for radio frequency ablation, the electrode comprising:
a cylindrical body (1) provided with first and second coupling grooves (1a, 1b) formed on an outer circumferential surface of the cylindrical body (1);
an active electrode body (2) inserted in the first coupling groove (1a) of the cylindrical body (1) and connected to a first terminal (51) of a radiofrequency generator (50); and
a passive electrode body (3) inserted in the second coupling groove (1b) of the cylindrical body (1) and connected to a second terminal (52) of the radiofrequency generator (50)
wherein the first and second coupling grooves (1a, 1b) are formed on an outer circumferential surface of the cylindrical body (1) in a screw thread shape and spaced apart from each other at a predetermined interval, the active electrode body (2) being wound on the first coupling groove (1a) a plurality of times and the passive electrode body (3) being wound on the second coupling groove (1b) a plurality of times, or
wherein the first and second coupling grooves (1a, 1b) are formed by extending in a lengthwise direction of the cylindrical body (1),
wherein the cylindrical body (1) is made of an insulating material and **characterised in that** the cylindrical body is provided with a cooling water circulation channel (1c) in which cooling water supplied from a cooling pipe (40) circulates, the cooling water circulation channel (1c) including a cylindrical inflow channel (1c') into which cooling water supplied from the cooling pipe (40) flows, and a cylindrical outflow channel (1c") provided outside the inflow channel (1c') and discharging the cooling water flowing in from the inflow channel (1c') to the cooling pipe (40).

2. The electrode (10) of claim 1, wherein the outflow channel (1c") and the inflow channel (1c') extend to a distal end of the cylindrical body (1) and the outflow channel (1c") has a length in the distal direction longer than that of the inflow channel (1c'), so that cooling water flowing into the inflow channel (1c') moves from the distal end to a proximal end of the cylindrical body (1) through the outflow channel (1c") and is then recovered again by the cooling pipe at the proximal end (40).

## Patentansprüche

1. Bipolare Elektrode (10) zur Radiofrequenzablation, wobei die Elektrode Folgendes umfasst:
einen zylindrischen Körper (1), der mit ersten und zweiten Kopplungsnuten (1a, 1b) versehen ist, die auf einer äußeren Umfangsfläche des zylindrischen Körpers (1) ausgebildet sind;
einen aktiven Elektrodenkörper (2), der in die erste Kopplungsnut (1a) des zylindrischen Körpers (1) eingesetzt und mit einem ersten Anschluss (51) eines Radiofrequenzgenerators (50) verbunden ist; und
einen passiven Elektrodenkörper (3), der in die zweite Kopplungsnut (1b) des zylindrischen Körpers (1) eingesetzt und mit einem zweiten Anschluss (52) des Radiofrequenzgenerators (50) verbunden ist,
wobei die erste und die zweite Kopplungsnuten (1a, 1b) auf einer äußeren Umfangsfläche des zylindrischen Körpers (1) schraubengewindeförmig ausgebildet sind und in einem vorbestimmten Abstand voneinander beabstandet sind, wobei der aktive Elektrodenkörper (2) eine Vielzahl von Malen auf die erste Kopplungsnut (1a) gewunden ist und der passive Elektrodenkörper (3) eine Vielzahl von Malen auf die zweite Kopplungsnut (1b) gewunden ist, oder
wobei die ersten und zweiten Kopplungsnuten (1a, 1b) durch Ausdehnung in einer Längsrichtung des zylindrischen Körpers (1) gebildet werden,
wobei
der zylindrische Körper (1) aus einem Isoliermaterial hergestellt ist und **dadurch gekennzeichnet ist, dass** der zylindrische Körper mit einem Kühlwasser-Umlaufkanal (1c) versehen ist, in dem von einem Kühlrohr (40) zugeführtes Kühlwasser umläuft, wobei der Kühlwasser-Umlaufkanal (1c) einen zylindrischen Einströmkanal (1c'), in den von dem Kühlrohr (40) zugeführtes Kühlwasser einströmt, und einen zylindrischen Ausströmkanal (1c") beinhaltet, der außerhalb des Einströmkanals (1c') vorgesehen ist und das von dem Einströmkanal (1c') einströmende Kühlwasser zu dem Kühlrohr (40) abführt.

2. Elektrode (10) gemäß Anspruch 1, wobei sich der Ausströmkanal (1c") und der Einströmkanal (1c') bis zu einem distalen Ende
des zylindrischen Körpers (1) erstrecken und der Ausströmkanal (1c") in distaler Richtung eine Länge hat, die länger ist als die des Einströmkanals (1c'), so dass sich das in den Einströmkanal (1c') einströmende Kühlwasser vom distalen Ende durch den Ausströmkanal (1c") zu einem proximalen Ende des zylindrischen Körpers (1) bewegt und dann durch das Kühlrohr am proximalen Ende (40) wieder zurückgewonnen wird.

## Revendications

1. Électrode bipolaire (10) pour ablation par radiofréquence, l'électrode comprenant :
un corps cylindrique (1) doté de première et seconde rainures de couplage (1a, 1b) formées sur une surface circonférentielle externe du corps cylindrique (1) ;
un corps d'électrode active (2) inséré dans la première rainure de couplage (1a) du corps cylindrique (1) et relié à une première borne (51) d'un générateur de radiofréquences (50) ; et
un corps d'électrode passive (3) inséré dans la seconde rainure de couplage (1b) du corps cylindrique (1) et relié à une seconde borne (52) du générateur de radiofréquences (50)
dans laquelle les première et seconde rainures de couplage (1a, 1b) sont formées sur une surface circonférentielle externe du corps cylindrique (1) suivant une forme de vis filetée et espacées l'une de l'autre à un intervalle prédéterminé, le corps d'électrode active (2) étant enroulé sur la première rainure de couplage (1a) une pluralité de fois et le corps d'électrode passive (3) étant enroulé sur la seconde rainure de couplage (1b) une pluralité de fois, ou
dans laquelle les première et seconde rainures de couplage (1a, 1b) sont formées par extension dans une direction de longueur du corps cylindrique (1),
dans laquelle
le corps cylindrique (1) est constitué d'un matériau isolant, et **caractérisée en ce que** le corps cylindrique est muni d'un canal de circulation d'eau de refroidissement (1c) dans lequel circule de l'eau de refroidissement fournie depuis un tuyau de refroidissement (40), le canal de circulation d'eau de refroidissement (1c) incluant un canal d'entrée cylindrique (1c') dans lequel circule l'eau de refroidissement fournie depuis le tuyau de refroidissement (40), et un canal de sortie cylindrique (1c") placé à l'extérieur du canal d'entrée (1c') et évacuant l'eau de refroidissement circulant depuis le canal d'entrée (1c') vers le tuyau de refroidissement (40).

2. Électrode (10) selon la revendication 1, dans laquelle le canal de sortie (1c") et le canal d'entrée (1c') s'étendent jusqu'à une extrémité distale du corps cylindrique (1) et le canal de sortie (1c") présente une longueur dans la direction distale supérieure à celle du canal d'entrée (1c'), de sorte que l'eau de refroidissement circulant dans le canal d'entrée (1c') se déplace de l'extrémité distale à une extrémité proximale du corps cylindrique (1) par l'intermédiaire du canal de sortie (1c") et est ensuite récupérée de nouveau par le tuyau de refroidissement à l'extrémité proximale (40).
